# DEMANDE DE BREVET EUROPEEN

(11) **EP 1 382 328 A1**
(43) Date de publication de la demande: **21.01.2004**
(21) Numéro de dépôt: 03291560.5
(22) Date de dépôt: 25.06.2003
(51) Int. Cl.: A61K 7/42

(54) **Matériau photostabilisé contenant un filtre solaire du type dibenzoylmethane, obtenu par voie sol-gel, et composition cosmétique et/ou dermatologique contenant un tel matériau**

(30) Priorité: 19.07.2002 FR 0209210
(71) Demandeur: L'OREAL, 75008 Paris (FR)
(72) Inventeur: Chodorowski-Kimes, Sandrine, 60300 Senlis (FR)
(74) Mandataire: Casalonga, Axel

(57) **Abrégé**

La présente invention concerne un procédé de photostabilisation d'un filtre solaire organique du type dibenzoylméthane par immobilisation dudit filtre dans une matrice élaborée par voie sol-gel à partir d'au moins un alcoxyde de silicium, d'au moins un tensioactif et d'eau, sans ajout de solvant organique. Elle concerne également un procédé de préparation d'un matériau contenant un filtre solaire organique du type dibenzoylméthane, le matériau susceptible d'être obtenu selon ce procédé de préparation et une composition cosmétique et/ou dermatologique comprenant un tel matériau.

## Description

La présente invention concerne un procédé de photostabilisation d'un filtre solaire organique du type dibenzoylméthane, et un procédé de préparation d'un matériau comprenant un tel filtre par voie sol-gel. Elle concerne aussi le matériau ainsi produit et une composition cosmétique et/ou dermatologique contenant un tel matériau.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connues sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A sont plus pénétrants que les rayons UV-B et provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc souhaitable de filtrer aussi le rayonnement UV-A, et en particulier le rayonnement UV-A long.

Ainsi, dans le but d'assurer une protection de la peau et des matières kératiniques contre le rayonnement UV, on utilise généralement des compositions antisolaires comprenant des filtres actifs dans l'UV-A et des filtres actifs dans l'UV-B.

Dans cette optique, une famille bien connue de filtres actifs dans l'UV-A et particulièrement intéressante est constituée par les dérivés du dibenzoylméthane et notamment, le 4-tert-butyl-4'-méthoxydibenzoylméthane commercialisé par la société HOFFMANN-LAROCHE sous la dénomination "PARSOL® 1789".

Or, les filtres solaires tels que le Parsol® 1789, présentent un problème majeur de sensibilité au rayonnement UV. En effet, il se trouve que les dérivés du dibenzoylméthane sont des produits relativement sensibles au rayonnement ultraviolet. Ils présentent une fâcheuse tendance à se dégrader plus ou moins rapidement sous l'action de ces derniers. Ainsi, cette sensibilité photochimique des dérivés du dibenzoylméthane face au rayonnement ultraviolet auquel ils sont par nature destinés à être soumis, ne permet pas de garantir une protection constante durant une exposition solaire prolongée, de sorte que, de façon contraignante, des applications répétées à intervalles de temps réguliers et rapprochés doivent être effectuées par l'utilisateur pour obtenir une protection efficace de la peau contre le rayonnement UV.

Par conséquent, on a cherché à réduire la sensibilité au rayonnement UV de ces filtres, mais on se heurte alors à de nombreux problèmes de formulation. Par exemple, ces filtres photosensibles particuliers peuvent ne pas être formulables avec certains autres produits actifs, nécessitant à chaque fois le développement d'une nouvelle formulation. Par ailleurs, ces filtres solaires photosensibles ont un effet protecteur nécessairement limité dans le temps et pour compenser leur sensibilité au rayonnement UV, il faut en inclure des quantités importantes dans les formulations sans que cela suffise pour autant à résoudre le problème.

Ce problème de photoinstabilité des filtres solaires du type dibenzoylméthane est bien connu de l'homme de métier.

En effet, la demande de brevet FR-A-2 799 119 décrit un procédé permettant de stabiliser vis-à-vis du rayonnement UV un filtre solaire photosensible de type dibenzoylméthane par son incorporation dans une matrice élaborée par voie sol-gel à partir d'au moins un alcoxyde de silicium, d'au moins un solvant, d'au moins un tensioactif et d'eau. Cependant, l'amélioration de la photostabilité de ces filtres n'est pas toujours satisfaisante.

La Demanderesse a alors trouvé de façon surprenante que l'immobilisation d'un filtre solaire organique du type dibenzoylméthane dans une matrice élaborée par voie sol-gel à partir d'au moins un alcoxyde de silicium, d'au moins un tensioactif et d'eau, sans ajout de solvant organique, conduisait à l'obtention d'un produit qui présente une meilleure photostabilité.

D'autres propriétés avantageuses sont également obtenues avec ce nouveau matériau solaire. En particulier, le matériau et les compositions le comprenant sont filmifiables, confèrent un toucher agréable et présentent une très bonne rémanence à l'eau, pure ou saline, et aux huiles cosmétiques polaires et apolaires.

La présente invention a donc pour objet un procédé de photostabilisation d'un filtre solaire organique du type dibenzoylméthane.

La présente invention a également pour objet un procédé de préparation d'un matériau contenant un filtre organique du type dibenzoylméthane par voie sol-gel.

Un autre objet consiste en un matériau susceptible d'être obtenu à partir du procédé de préparation selon l'invention.

La présente invention a encore pour objet une composition cosmétique et/ou dermatologique comprenant un tel matériau.

D'autres caractéristiques, aspects et avantages de l'invention apparaîtront encore plus clairement à la lecture de la description et des divers exemples qui suivent.

Le procédé de photostabilisation d'un filtre solaire organique du type dibenzoylméthane selon la présente invention, consiste à immobiliser ledit filtre dans une matrice élaborée par voie sol-gel à partir d'au moins un alcoxyde de silicium, d'au moins un tensioactif et d'eau, sans ajout de solvant organique.

On entend par filtre solaire organique du type dibenzoylméthane, tout composé sous forme de molécule simple, dimère, oligomère ou polymère, possédant au moins un groupe chromophore dibenzoylméthane.

Parmi les filtres solaires organiques du type dibenzoylméthane pouvant être utilisés dans le cadre de la présente invention, on peut notamment citer, de manière non limitative :
le 2-méthyldibenzoylméthane,
le 4-méthyldibenzoylméthane,
le 4-isopropyldibenzoylméthane ou Isopropyl Dibenzoylmethane, tel que celui vendu sous la dénomination "EUSOLEX® 8020" par la société MERCK,
le 4-tert-butyldibenzoylméthane,
le 2,4-diméthyldibenzoylméthane,
le 2,5-diméthyldibenzoylméthane,
le 4,4'-diisopropyldibenzoylméthane,
le 4,4'-diméthoxydibenzoylméthane,
le 4-tert-butyl-4'-méthoxydibenzoylméthane ou Butyl Methoxydibenzoylmethane,
le 2-méthyl-5-isopropyl-4'-méthoxydibenzoylméthane,
le 2-méthyl-5-tert-butyl-4'-méthoxydibenzoylméthane,
le 2,4-diméthyl-4'-méthoxydibenzoylméthane, et
le 2,6-diméthyl-4-tert-butyl-4'-méthoxydibenzoylméthane.

Comme filtre solaire organique du type dibenzoylméthane, on utilise de préférence le Butyl Methoxydibenzoylmethane vendu notamment sous la dénomination commerciale "PARSOL® 1789" par la société HOFFMANN-LA ROCHE, répondant à la formule développée suivante :

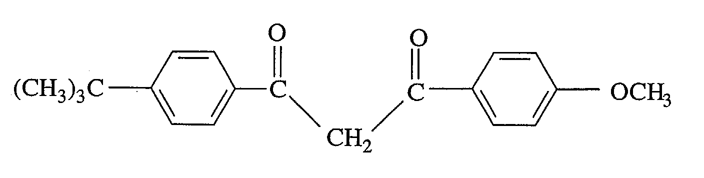

Les alcoxydes de silicium utilisés dans le procédé selon la présente invention sont notamment choisis parmi ceux répondant aux formules suivantes :

Si(ORₐ)₄ (I)

R-Si(ORₐ)₃ (II)

(RₐO)₃-Si-R"-Si(ORₐ)₃ (III)

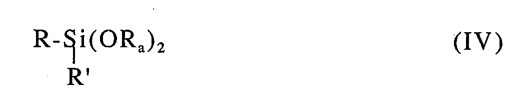

dans lesquelles :
Rₐ représente un groupe alkyle linéaire ou ramifié, en C₁₋₃₀, de préférence en C₁₋₆;
R et R', indépendamment l'un de l'autre, représentent un groupe alkyle linéaire ou ramifié, un groupe cycloalkyle, un groupe aryle substitué ou non, lesdits groupes R et R' pouvant comporter en plus un groupe cosmétiquement ou dermatologiquement actif ;
R" représente un groupe alkylène linéaire ou ramifié, un groupe cycloalkylène, un groupe arylène substitué ou non, ledit groupe R" pouvant comporter en plus un groupe cosmétiquement ou dermatologiquement actif.

Parmi les groupes cosmétiquement ou dermatologiquement actifs qui peuvent être portés par les groupes R, R' et R", on peut mentionner, par exemple, un groupe colorant, un groupe photochromique, un groupe favorisant l'adhérence sur les matières kératiniques comme des groupes de type phoshates, phosphonates, acide phosphonique, amide, uréthanne, uréido, hydroxy, carboxy, thiol, acide aminé ou polypeptide, un groupe anti-radicaux libres ou un groupe porteur de vitamine.

Parmi les alcoxydes de silicium définis ci-dessus, on préfère tout particulièrement le tétraéthoxysilane et le méthyltriéthoxysilane.

Les tensioactifs utilisés dans le procédé selon la présente invention sont de préférence non ioniques. Ils sont notamment choisis parmi les alcools, les alkylphénols ou les acides gras polyéthoxylés, polypropoxylés ou polyglycérolés, à chaîne grasse comportant de 6 à 32 atomes de carbone, de préférence de 8 à 18 atomes de carbone, le nombre de groupements oxyde d'éthylène ou oxyde de propylène étant compris entre 2 et 100, de préférence entre 2 et 50 et le nombre de groupements glycérol étant compris entre 2 et 30.

On peut également citer comme tensioactifs non ioniques, les copolymères d'oxydes d'éthylène et de propylène; les condensats d'oxydes d'éthylène et de propylène sur des alcools gras ; les amides gras polyéthoxylés ayant de préférence 2 à 30 moles d'oxyde d'éthylène ; les amides gras polyglycérolés comportant de préférence 1 à 5 groupements glycérol et en particulier 1,5 à 4 ; les amines grasses polyéthoxylées ayant de préférence 2 à 30 moles d'oxyde d'éthylène ; les esters d'acides gras du sorbitanne éthoxylés avec 2 à 30 moles d'oxyde d'éthylène ; les esters d'acide gras de saccharose, les esters d'acides gras du polyéthylèneglycol, les alkylpolyglucosides, les dérivés carbamates ou amides de N-alkylglucamines, les aldobionamides, les oxydes d'amines tels que les oxydes d'alkylamines ou de N-acylamidopropylmorpholine.

Parmi les tensioactifs mentionnés ci-dessus, on préfère utiliser en particulier un 4-(1,1,3,3-tétraméthylbutyl)phénol polyéthoxylé. Ce dernier est commercialisé sous le nom de Triton® X100 chez Union Carbide.

Un autre objet de l'invention consiste en un procédé de préparation d'un matériau contenant un filtre solaire organique du type dibenzoylméthane, par voie sol-gel, consistant à mélanger au moins un filtre solaire organique du type dibenzoylméthane, au moins un tensioactif, au moins un alcoxyde de silicium et de l'eau en une quantité suffisante pour l'hydrolyse partielle ou totale de l'alcoxyde et sa condensation, en l'absence de solvant organique.

Les filtres solaires organiques du type dibenzoylméthane, les tensioactifs, et les alcoxydes de silicium utilisés dans ledit procédé de préparation sont tels que décrits ci-dessus.

On choisira pour un filtre solaire organique donné, du type dibenzoylméthane, les composants de départ, c'est-à-dire les tensioactifs et les alcoxydes de silicium, ainsi que leurs quantités et la quantité d'eau de manière à ce que ledit filtre soit dispersé de manière homogène dans le matériau final.

Lesdits filtres solaires sont notamment utilisés dans le procédé de la présente invention en une quantité allant de préférence de 0,1 % à 20 % en poids, mieux encore de 0,5 à 10 % en poids par rapport au poids de l'alcoxyde de silicium.

La quantité de tensioactif utilisée dans le cadre de l'invention dépend de la solubilité du tensioactif dans le mélange alcoxyde/eau. Elle est telle que le rapport pondéral du tensioactif à l'alcoxyde de silicium est notamment compris entre 0,01 et 99, et de préférence entre 1 et 60.

L'eau est utilisée en une quantité suffisante pour l'hydrolyse totale ou partielle de l'alcoxyde de silicium et sa condensation. Cette quantité est comprise entre 0,01 et 99 % en poids, de préférence entre 1 et 50 % en poids par rapport au poids de l'alcoxyde de silicium.

L'eau comprend de préférence un acide minéral ou organique, cosmétiquement acceptable, et plus particulièrement l'acide chlorhydrique, l'acide nitrique, l'acide sulfurique ou l'acide acétique. Dans le cadre de la présente invention, on préfère tout particulièrement l'acide chlorhydrique.

Le pH de l'eau de l'hydrolyse se situe de préférence dans l'intervalle allant de 1 à 4, mieux encore de 1 à 2.

Un mode de réalisation préféré du procédé de préparation défini ci-dessus consiste à ajouter au mélange d'au moins un tensioactif, de préférence non ionique, et d'un filtre solaire du type dibenzoylméthane, dans un premier temps l'alcoxyde de silicium, et dans un second temps l'eau contenant un acide minéral ou organique, cosmétiquement acceptable.

L'invention concerne également le matériau contenant un filtre solaire organique du type dibenzoylméthane, susceptible d'être obtenu selon le procédé de préparation décrit ci-dessus.

Un objet supplémentaire de la présente invention est une composition cosmétique et/ou dermatologique comprenant, dans un milieu cosmétiquement et/ou dermatologiquement acceptable, une quantité efficace d'au moins un matériau contenant un filtre solaire organique du type dibenzoylméthane, susceptible d'être obtenu selon le procédé de préparation de la présente invention.

Par milieu cosmétiquement et/ou dermatologiquement acceptable, on entend un milieu compatible avec toutes les matières kératiniques telles que la peau, les cheveux, les ongles, les cils et sourcils, les lèvres et toute autre zone du corps et du visage.

Cette composition cosmétique et/ou dermatologique comprend ledit matériau en une quantité efficace allant de 1 % en poids à 99 % en poids, de préférence de 1 % en poids à 60 % en poids, par rapport au poids total de la composition.

De préférence, le matériau selon l'invention est séché et broyé, et ajouté sous forme de particules dans une composition cosmétique et/ou dermatologique. La taille moyenne des particules dépend du type de la méthode de broyage et de l'appareillage utilisés et elle se situe avantageusement dans l'intervalle allant de 0,1 à 50 µm, de préférence de 0,1 à 20 µm, et mieux encore de 0,1 à 10 µm.

Les compositions cosmétiques et/ou dermatologiques selon la présente invention peuvent contenir en outre un ou plusieurs filtres solaires organiques complémentaires actifs dans l'UV-A et/ou l'UV-B, hydrosolubles, liposolubles, ou insolubles dans les solvants cosmétiques usuels, différents des filtres solaires du type dibenzoylméthane définis ci-dessus, sous réserve bien entendu qu'ils n'altèrent pas la caractéristique essentielle de ces compositions, à savoir leur photostabilité.

Ces filtres complémentaires peuvent être notamment choisis parmi les anthranilates ; les dérivés salicyliques ; les dérivés cinnamiques ; les dérivés du camphre ; les dérivés de triazine tels que ceux décrits dans les demandes de brevet US 4 367 390, EP0863145, EP0517104, EP0570838, EP0796851, EP0775698, EP0878469 et EP0933376 ; les dérivés de la benzophénone ; les 2-hydroxybenzophénones aminées telles que celles décrites dans les demandes de brevets EP 1 046 391 et DE 1 00 12408 ; les dérivés de β,β'-diphénylacrylate ; les dérivés de benzotriazole ; les dérivés de benzalmalonate ; les dérivés de benzimidazole ; les imidazolines ; les dérivés de bis(benzoazolyle) tels que ceux décrits dans la demande de brevet EP0669323 et US 2 463 264 ; les dérivés de l'acide p-aminobenzoïque (PABA) ; les dérivés de méthylène-bis(hydroxyphénylbenzotriazole) tels que ceux décrits dans les demandes de brevet US 5 237 071, US 5 166 355, GB2303549, DE19726184 et EP0893119; les polymères hydrocarbonés filtres et silicones filtres décrits dans la demande WO-93/04665 ; les dérivés d'alkylstyrène tels que ceux décrits dans la demande de brevet DE 198 55 649 ; et leurs mélanges.

On peut citer à titre d'exemples de tels filtres solaires organiques complémentaires actifs dans l'UV-A et/ou l'UV-B, les composés suivants désignés par leur nom INCI, ainsi que leur mélanges :

### anthranilates :

- Menthyl Anthranilate vendu sous le nom commercial NEO HELIOPAN® MA par HAARMAN ET REIMER ;

### dérivés salicyliques :

- Homosalate vendu sous le nom commercial EUSOLEX® HMS par RONA/EM INDUSTRIES,
- Ethylhexyl Salicylate vendu sous le nom commercial NEO HELIOPAN® OS par HAARMANN ET REIMER,
- Dipropyleneglycol Salicylate vendu sous le nom commercial DIPSAL® par SCHER,
- TEA Salicylate vendu sous le nom commercial NEO HELIOPAN® TS par HAARMANN ET REIMER ;

### dérivés cinnamiques

- Ethylhexyl Methoxycinnamate vendu notamment sous le nom commercial PARSOL® MCX par HOFFMANN LA ROCHE,
- Isopropyl Methoxycinnamate,
- Isoamyl Methoxycinnamate vendu sous le nom commercial NEO HELIOPAN® E 1000 par HAARMANN ET REIMER,
- Cinnoxate,
- DEA Methoxycinnamate,
- Diisopropyl Methylcinnamate,
- Glyceryl Ethylhexanoate Dimethoxycinnamate ;

### dérivés du camphre :

- 3-Benzylidene Camphor fabriqué sous le nom MEXORYL® SD par CHIMEX,
- Benzylidene Camphor Sulfonic Acid fabriqué sous le nom MEXORYL® SL par CHIMEX,
- Camphor Benzalkonium Methosulfate fabriqué sous le nom MEXORYL® SO par CHIMEX,
- Terephthalylidene Dicamphor Sulfonic Acid fabriqué sous le nom MEXORYL® SX par CHIMEX ;
- Polyacrylamidomethyl Benzylidene Camphor fabriqué sous le nom MEXORYL® SW par CHIMEX,

### dérivés de triazine:

- Anisotriazine vendue sous le nom commercial TINOSORB® S par CIBA GEIGY,
- Ethylhexyl triazone vendue notamment sous le nom commercial UVINUL® T150 par BASF,
- Diéthylhexyl Butamido Triazone vendue sous le nom commercial UVASORB® HEB par SIGMA 3V,
- la 2,4,6-tris-(4'-aminobenzalmalonate de diisobutyle)-s-triazine ;

### dérivés de benzophénone :

- Benzophenone-1 vendue sous le nom commercial UVINUL® 400 par BASF,
- Benzophenone-2 vendue sous le nom commercial UVINUL® D-50 par BASF,
- Benzophenone-3 ou Oxybenzone vendue sous le nom commercial UVINUL® M-40 par BASF,
- Benzophenone-4 vendue sous le nom commercial UVINUL® MS-40 par BASF,
- Benzophenone-5,
- Benzophenone-6 vendue sous le nom commercial HELISORB® 11 par NORQUAY,
- Benzophenone-8 vendue sous le nom commercial SPECTRA-SORB® UV-24 par AMERICAN CYANAMID
- Benzophenone-9 vendue sous le nom commercial UVINUL® DS-49 par BASF,
- Benzophénone-12,
- le 2-(4-diéthylamino-2-hydroxybenzoyl)benzoate de n-hexyle ;

### dérivés de ββ'-diphénylacrylate:

- Octocrylene vendu notamment sous le nom commercial UVINUL® N539 par BASF,
- Ethocrylene, vendu notamment sous le nom commercial UVINUL® N35 par BASF ;

### dérivés de benzotriazole :

- Drometrizole Trisiloxane vendu sous le nom commercial SILATRIZOLE® par RHODIA CHIMIE,
- Methylene bis-Benzotriazolyl Tetramethylbutylphenol vendu sous forme solide sous le nom commercial MIXXIM® BB/100 par FAIRMOUNT CHEMICAL ou sous forme micronisée en dispersion aqueuse sous le nom commercial TINOSORB® M par CIBA SPECIALTY CHEMICALS ;

### dérivés de benzalmalonate :

- Polyorganosiloxane à fonctions benzalmalonate vendu sous le nom commercial PARSOL® SLX par HOFFMANN LAROCHE ;

### dérivés de benzimidazole :

- Phenylbenzimidazole Sulfonic Acid vendu notamment sous le nom commercial EUSOLEX® 232 par MERCK,
- Disodium Phenyl Dibenzimidazole Tetra-sulfonate vendu sous le nom commercial NEO HELIOPAN® AP par HAARMANN ET REIMER ;

### dérivés d'imidazoline :

- Ethylhexyl Dimethoxybenzylidene Dioxoimidazoline Propionate ;

### dérivés d'acide para-aminobenzoïque :

- PABA,
- Ethyl PABA,
- Ethyl Dihydroxypropyl PABA,
- Ethylhexyl Dimethyl PABA vendu notamment sous le nom commercial ESCALOL® 507 par ISP,
- Glyceryl PABA,
- PEG-25 PABA vendu sous le nom commercial UVINUL® P25 par BASF.

Les filtres UV organiques complémentaires plus particulièrement préférés sont choisis parmi les composés suivants :
- Ethylhexyl Salicylate,
- Octocrylene,
- Phenylbenzimidazole Sulfonic Acid,
- Terephthalylidene Dicamphor Sulfonic Acid,
- 4-Methylbenzylidene camphor,
- Anisotriazine,
- Ethylhexyl Triazone,
- Diethylhexyl Butamido Triazone,
- la 2,4,6-tris-(4'-amino benzalmalonate de diisobutyle)-s- triazine,
- Benzophenone-3,
- Benzophenone-4,
- Benzophenone-5,
- 2-(4-diéthylamino-2-hydroxybenzoyl)benzoate de n-hexyle ;
- Methylene bis-Benzotriazolyl Tetramethylbutylphenol,
- Drometrizole Trisiloxane,
- Disodium Phenyl Dibenzimidazole Tetra-sulfonate,
et leurs mélanges.

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non, comme par exemple, des nanopigments d'oxyde de titane (amorphe ou cristallisé sous forme rutile et/ou anatase), de fer, de zinc, de zirconium ou de cérium qui sont tous des agents photoprotecteurs bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0 518 772 et EP-A-0 518 773.

Les compositions selon l'invention peuvent également contenir des agents de bronzage et/ou de brunissage artificiel de la peau (agents autobronzants) tels que, par exemple, de la dihydroxyacétone (DHA).

Les compositions conformes à la présente invention peuvent comprendre en outre des adjuvants cosmétiques classiques, notamment choisis parmi les corps gras, les solvants organiques, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs anioniques, cationiques, amphotères ou non ioniques, les anti-inflammatoires, les antagonistes de substance P, les charges, les séquestrants, les polymères, les propulseurs, les agents alcalinisants ou acidifiants, les colorants ou tout autre ingrédient habituellement utilisé dans le domaine cosmétique et/ou dermatologique, en particulier pour la fabrication de compositions antisolaires sous forme d'émulsions, et leurs mélanges.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35 °C. Ils comprennent également les acides gras, les alcools gras et les esters d'acides gras, linéaires ou cycliques tels que les dérivés d'acide benzoïque, trimellitique et hydroxybenzoïque.

Comme huiles, on peut citer les huiles minérales (vaseline) ; végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination "Finsolv TN" par la société Finetex, le palmitate d'octyle, le lanolate d'isopropyle, les triglycérides dont ceux des acides caprique/caprylique), les esters et éthers gras éthoxylés ou propoxylés ; siliconées (cyclométhicone de préférence à 4 ou 5 atomes de silicium, polydiméthylsiloxane ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxy-éthylcellulose, l'hydroxypropylméthylcellulose et les gommes de silicone comme, par exemple, un dérivé de polydiméthylsiloxane.

Les compositions cosmétiques et/ou dermatologiques selon l'invention peuvent être préparées selon les techniques bien connues de l'homme de métier, en particulier celles destinées à la préparation d'émulsions de type huile-dans-l'eau ou eau-dans-l'huile

Ces compositions cosmétiques et/ou dermatologiques peuvent se présenter en particulier sous la forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins, J. Mol. Biol. 13, 238 (1965), FR 2 315 991 e FR 2 416 008).

La composition cosmétique et/ou dermatologique selon l'invention peut être utilisée comme composition protectrice des matières kératiniques telles que la peau, les cheveux, les ongles, les lèvres, les cils ou les sourcils, contre les rayons ultraviolets, comme composition antisolaire ou comme produit de maquillage.

Lorsque la composition cosmétique selon l'invention est utilisée pour la protection de la peau contre les rayons UV ou comme composition antisolaire, elle peut se présenter sous forme de suspension ou de dispersion dans des solvants ou des corps gras, sous forme de dispersion vésiculaire non ionique, ou encore d'émulsion, de préférence, de type huile-dans-eau, telle qu'une crème ou un lait, ou sous forme de pommade, de gel, de gel crème, de bâtonnet solide, de poudre, de stick, de mousse aérosol ou de spray.

Lorsque la composition cosmétique selon l'invention est utilisée pour la protection des cheveux, elle peut se présenter sous forme de shampoing, de lotion, de gel, d'émulsion, de dispersion vésiculaire non ionique, et peut constituer, par exemple, une composition à rincer, à appliquer avant ou après shampoing, avant ou après coloration ou décoloration, avant, pendant ou après permanente ou défrisage, une lotion ou un gel coiffant ou traitant, une lotion ou un gel pour le brushing ou la mise en plis, une composition de permanente ou de défrisage, de coloration ou décoloration des cheveux.

Lorsque la composition cosmétique selon l'invention est utilisée comme produit de maquillage des cils, des sourcils ou de la peau, tel que crème de traitement de l'épiderme, fond de teint, bâton de rouge à lèvre, fard à paupières, fard à joues, mascara ou "ligneur" encore appelé "eye-liner", elle peut se présenter sous forme solide ou pâteuse, anhydre ou aqueuse, comme des émulsions de type huile-dans-eau ou eau-dans-huile, des dispersions vésiculaires non ioniques ou encore des dispersions.

Les exemples suivants sont donnés à titre illustratif de la présente invention.

### Exemple 1

On prépare un matériau par voie sol-gel à partir des ingrédients suivants :

| | |
|---|---|
| Tétraéthoxysilane (TEOS) | 5,0 g |
| Eau (HCl 0, 1 N) | 0,87 g |
| Triton® X-100 | 0,75 g |
| Parsol® 1789 | 0,25 g |

le tétraéthoxysilane provenant de la société Fluka, le Parsol® 1789 étant commercialisé par la société HOFFMANN-LAROCHE, et le Triton® X-100 étant commercialisé par la société Union Carbide.

On disperse le Parsol® 1789 dans le Triton® X-100 en chauffant à 40 °C pendant 30 minutes. On ajoute ensuite le TEOS à cette dispersion sous agitation à température ambiante. Puis, on ajoute goutte à goutte l'eau acidifiée. On agite le tout pendant 24 heures à température ambiante. On obtient un matériau prêt à l'emploi.

### Exemple comparatif

On prépare un matériau par voie sol-gel à partir des ingrédients suivants :

| | |
|---|---|
| Tétraéthoxysilane (TEOS) | 5,0 g |
| Ethanol | 17 ml |
| Eau (HCl 0, 1 N) | 0,9 g |
| Triton® X-100 | 0,75 g |
| Parsol® 1789 | 0,25 g |

le tétraéthoxysilane provenant de la société Fluka, le Parsol® 1789 étant commercialisé par la société HOFFMANN-LAROCHE, et le Triton® X-100 étant commercialisé par la société Union Carbide.

On disperse le Parsol® 1789 dans le Triton® X-100 en chauffant à 40 °C pendant 30 minutes. On ajoute ensuite le TEOS/éthanol à la dispersion sous agitation à température ambiante. Puis, on ajoute goutte à goutte l'eau acidifiée. On agite le tout pendant 24 heures à température ambiante. On obtient un matériau prêt à l'emploi.

### Essais de stabilité vis-à-vis du rayonnement UV

On a ensuite réalisé un essai de stabilité vis-à-vis du rayonnement UV du Parsol® 1789 en irradiant le matériau obtenu dans l'exemple 1 ci-dessus ainsi que, à titre de comparaison, du matériau obtenu dans l'exemple comparatif.

On a préparé six plaques de verre (lames de microscope) en déposant sur chacune, 100 µl du matériau de l'exemple 1. Après évaporation des composants volatils du mélange, on a obtenu des films. Le temps de séchage des films est rapide, c'est-à-dire entre 1 et 3 minutes. Le matériau est néanmoins laissé tel quel à température ambiante pendant 24 heures, avant irradiation.

On a alors irradié trois des films obtenus en utilisant un UVIMETRE OSTRAM-Centra équipé d'une lampe au xénon, dans les conditions suivantes :
- Energie de la lampe UV-A = 22 J/cm²,
- Temps d'irradiation du simulateur = 20 minutes.

Ces conditions correspondent à environ 1 heure d'ensoleillement UV-A solaire.

On a évalué la photodégradation du Parsol® 1789 par mesure de la quantité de Parsol® 1789 avant et après irradiation : on a extrait le Parsol® 1789 des films de chacune des trois plaques non irradiées et des trois plaques irradiées avec un volume déterminé d'éthanol en y laissant les films pendant 24 heures et en agitant de temps en temps. De cette manière, on a extrait tout le Parsol® 1789 présent dans les films avant et après l'irradiation, puis on a mesuré la quantité de Parsol® 1789.

Les résultats obtenus pour le matériau de l'exemple 1 sont regroupés dans le tableau 1 suivant :

**Tableau 1**

| **Matériau de l'ex. 1** | m_{Parsol1789} (x10⁻³) g | | | | | | % de dégradation du Parsol® 1789 |
|---|---|---|---|---|---|---|---|
| | Plaque n° | | | | | | |
| | 1 | 2 | 3 | 4 | 5 | 6 | |
| Avant irradiation | 3.1 | 3.4 | 3.1 | | | | |
| Après irradiation | | | | 3.1 | 2.8 | 3 | 7% |

On a évalué la photodégradation du Parsol® 1789 dans le matériau de l'exemple comparatif en opérant de la même manière que pour le matériau de l'exemple 1. Les résultats obtenus sont regroupés dans le tableau 2 suivant :

**Tableau 2**

| **Matériau de l'ex. camp.** | m_{Parsol1789} (x10⁻⁴) g | | | | | | % de dégradation du Parsol® 1789 |
|---|---|---|---|---|---|---|---|
| | Plaque n° | | | | | | |
| | 1 | 2 | 3 | 4 | 5 | 6 | |
| Avant irradiation | 6.1 | 8 | 7.1 | | | | |
| Après irradiation | | | | 4.8 | 4.5 | 4.6 | 34% |

D'après les résultats ci-dessus, on obtient une photodégradation nettement moins importante (de l'ordre de 7%) pour le matériau selon l'invention, que celle obtenue avec le matériau de l'exemple comparatif (de l'ordre de 34%).

On a également observé en ce qui concerne le matériau de l'exemple 1 :
- que la qualité du film n'est pas altérée par exposition au soleil ;
- un faible relargage du Parsol® 1789 dans l'eau et dans une huile cosmétique, comme l'huile d'abricot et l'huile de Parléam ; et
- une bonne rémanence à l'eau du film.

## Revendications

1. Procédé de photostabilisation d'un filtre solaire organique du type dibenzoylméthane par immobilisation dudit filtre dans une matrice élaborée par voie sol-gel à partir d'au moins un alcoxyde de silicium, d'au moins un tensioactif non ionique et d'eau, sans ajout de solvant organique.

2. Procédé de photostabilisation selon la revendication 1, **caractérisé en ce que** le filtre solaire organique du type dibenzoylméthane est choisi parmi :
le 2-méthyldibenzoylméthane,
le 4-méthyldibenzoylméthane,
le 4-isopropyldibenzoylméthane,
le 4-tert-butyldibenzoylméthane,
le 2,4-diméthyldibenzoylméthane,
le 2,5-diméthyldibenzoylméthane,
le 4,4'-diisopropyldibenzoylméthane,
le 4,4'-diméthoxydibenzoylméthane,
le 4-tert-butyl-4'-méthoxydibenzoylméthane,
le 2-méthyl-5-isopropyl-4'-méthoxydibenzoylméthane,
le 2-méthyl-5-tert-butyl-4'-méthoxydibenzoylméthane,
le 2,4-diméthyl-4'-méthoxydibenzoylméthane, et
le 2,6-diméthyl-4-tert-butyl-4'-méthoxydibenzoylméthane.

3. Procédé selon la revendication 2, **caractérisé en ce que** le filtre solaire organique du type dibenzoylméthane est le 4-tert-butyl-4'-méthoxydibenzoylméthane.

4. Procédé selon l'une quelconque des revendications précédentes, **caractérisé en ce que** l'alcoxyde de silicium est choisi parmi :
Si(ORₐ)₄ (I)
R-Si(ORₐ)₃ (II)
(RₐO)₃-Si-R"-Si(ORₐ)₃ (III) 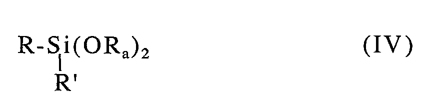
dans lesquelles :
Rₐ représente un groupe alkyle linéaire ou ramifié, en C₁₋₃₀, de préférence en C₁₋₆ ;
R et R', indépendamment l'un de l'autre, représentent un groupe alkyle linéaire ou ramifié, un groupe cycloalkyle, un groupe aryle substitué ou non, lesdits groupes R et R' pouvant comporter en plus un groupe cosmétiquement ou dermatologiquement actif ;
R" représente un groupe alkylène linéaire ou ramifié, un groupe cycloalkylène, un groupe arylène substitué ou non, ledit groupe R" pouvant comporter en plus un groupe cosmétiquement ou dermatologiquement actif.

5. Procédé selon la revendication 4, **caractérisé en ce que** l'alcoxyde de silicium est choisi parmi le tétra-éthoxysilane et le méthyltriéthoxysilane.

6. Procédé selon l'une quelconque des revendications précédentes, **caractérisé en ce que** le tensioactif est un tensioactif non ionique choisi parmi les alcools, les alkylphénols ou les acides gras polyéthoxylés, polypropoxylés ou polyglycérolés, à chaîne grasse comportant de 6 à 32 atomes de carbone, de préférence de 8 à 18 atomes de carbone, le nombre de groupements oxyde d'éthylène ou oxyde de propylène étant compris entre 2 et 100, de préférence entre 2 et 50 et le nombre de groupements glycérol étant compris entre 2 et 30.

7. Procédé selon la revendication 6, **caractérisé en ce que** le tensioactif est un 4-(1,1,3,3-tétraméthylbutyl)phénol polyéthoxylé.

8. Procédé de préparation d'un matériau contenant un filtre solaire organique du type dibenzoylméthane par voie sol-gel, consistant à mélanger au moins un filtre solaire organique du type dibenzoylméthane, au moins un tensioactif non ionique, au moins un alcoxyde de silicium et de l'eau en une quantité suffisante pour l'hydrolyse partielle ou totale de l'alcoxyde et sa condensation, en l'absence de solvant organique.

9. Procédé de préparation selon la revendication 8, **caractérisé en ce que** le filtre solaire organique du type dibenzoylméthane est choisi parmi :
le 2-méthyldibenzoylméthane,
le 4-méthyldibenzoylméthane,
le 4-isopropyldibenzoylméthane,
le 4-tert-butyldibenzoylméthane,
le 2,4-diméthyldibenzoylméthane,
le 2,5-diméthyldibenzoylméthane,
le 4,4'-diisopropyldibenzoylméthane,
le 4,4'-diméthoxydibenzoylméthane,
le 4-tert-butyl-4'-méthoxydibenzoylméthane,
le 2-méthyl-5-isopropyl-4'-méthoxydibenzoylméthane,
le 2-méthyl-5-tert-butyl-4'-méthoxydibenzoylméthane,
le 2,4-diméthyl-4'-méthoxydibenzoylméthane, et
le 2,6-diméthyl-4-tert-butyl-4'-méthoxydibenzoylméthane.

10. Procédé de préparation selon la revendication 9, **caractérisé en ce que** le filtre solaire organique du type dibenzoylméthane est le 4-tert-butyl-4'-méthoxydibenzoylméthane.

11. Procédé de préparation selon l'une quelconque des précédentes 8 à 10, **caractérisé en ce que** le filtre solaire organique du type dibenzoylméthane est présent en une quantité allant de 0,1 % à 20 % en poids, de préférence de 0,5 à 10 % en poids par rapport au poids de l'alcoxyde de silicium.

12. Procédé de préparation selon l'une quelconque des revendications 8 à 11, **caractérisé en ce que** l'alcoxyde de silicium est choisi parmi :
Si(ORₐ)₄ (I)
R-Si(ORₐ)₃ (II)
(RₐO)₃-Si-R"-Si(ORₐ)₃ (III) 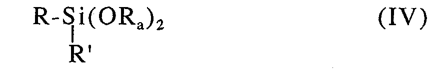
dans lesquelles :
Rₐ représente un groupe alkyle linéaire ou ramifié, en C₁₋₃₀, de préférence en C₁₋₆ ;
R et R', indépendamment l'un de l'autre, représentent un groupe alkyle linéaire ou ramifié, un groupe cycloalkyle, un groupe aryle substitué ou non, lesdits groupes R et R' pouvant comporter en plus un groupe cosmétiquement ou dermatologiquement actif ;
R" représente un groupe alkylène linéaire ou ramifié, un groupe cycloalkylène, un groupe arylène substitué ou non, ledit groupe R" pouvant comporter en plus un groupe cosmétiquement ou dermatologiquement actif.

13. Procédé de préparation selon la revendication 12, **caractérisé en ce que** l'alcoxyde de silicium est choisi parmi le tétraéthoxysilane et le méthyltriéthoxysilane.

14. Procédé de préparation selon l'une quelconque des revendications 8 à 13, **caractérisé en ce que** le tensioactif est un tensioactif non ionique choisi parmi les alcools, les alkylphénols ou les acides gras polyéthoxylés, polypropoxylés ou polyglycérolés, à chaîne grasse comportant de 6 à 32 atomes de carbone, de préférence de 8 à 18 atomes de carbone, le nombre de groupements oxyde d'éthylène ou oxyde de propylène étant compris entre 2 et 100, de préférence entre 2 et 50 et le nombre de groupements glycérol étant compris entre 2 et 30.

15. Procédé de préparation selon la revendication 14, **caractérisé en ce que** le tensioactif non ionique est un 4-(1,1,3,3-tétraméthylbutyl)phénol polyéthoxylé.

16. Procédé de préparation selon l'une quelconque des revendications 8 à 15, **caractérisé en ce que** le rapport pondéral du tensioactif à l'alcoxyde de silicium est compris entre 0,01 et 99, et de préférence entre 1 et 60.

17. Procédé de préparation selon l'une quelconque des précédentes 8 à 16, **caractérisé en ce que** l'eau contient un acide minéral ou organique, cosmétiquement acceptable.

18. Procédé de préparation selon l'une quelconque des précédentes 8 à 17, **caractérisé en ce que** l'eau de l'hydrolyse présente un pH compris entre 1 et 4, de préférence entre 1 et 2.

19. Procédé de préparation selon l'une quelconque des revendications 8 à 18, **caractérisé en ce que** l'on ajoute à un mélange d'au moins un tensioactif non ionique et d'un filtre solaire du type dibenzoylméthane, dans un premier temps au moins un alcoxyde de silicium, et dans un second temps de l'eau contenant un acide minéral ou organique cosmétiquement acceptable.

20. Matériau contenant un filtre solaire organique du type dibenzoylméthane, susceptible d'être obtenu par le procédé selon l'une quelconque des revendications 8 à 19.

21. Composition cosmétique et/ou dermatologique, comprenant dans un milieu cosmétiquement et/ou dermatologiquement acceptable, une quantité efficace d'au moins un matériau selon la revendication 20.

22. Composition selon la revendication 21, **caractérisée en ce que** le matériau est présent en une quantité de 1 à 99 % en poids, de préférence de 1 à 60 % en poids, par rapport au poids total de la composition.

23. Composition selon la revendication 21 ou 22, **caractérisée en ce qu'**elle comprend en outre un ou plusieurs filtres solaires organiques complémentaires actifs dans l'UV-A et/ou l'UV-B choisis parmi les anthranilates ; les dérivés salicyliques ; les dérivés cinnamiques ; les dérivés du camphre ; les dérivés de triazine; les dérivés de la benzophénone ; les 2-hydroxybenzophénones aminées ; les dérivés de β,β'-diphénylacrylate; les dérivés de benzotriazole ; les dérivés de benzalmalonate ; les dérivés de benzimidazole ; les imidazolines ; les dérivés de bis(benzoazolyle) ; les dérivés de l'acide p-aminobenzoïque (PABA) ; les dérivés de méthylène-bis(hydroxyphénylbenzotriazole) ; les polymères hydrocarbonés filtres et silicones filtres ; les dérivés d'alkylstyrène ; et leurs mélanges.

24. Composition selon la revendication 23, **caractérisée en ce que** le filtre solaire organique complémentaire actif dans l'UV-A et/ou l'UV-B est choisi parmi :
- Ethylhexyl Salicylate,
- Octocrylene,
- Phenylbenzimidazole Sulfonic Acid,
- Terephthalylidene Dicamphor Sulfonic Acid,
- 4-Methylbenzylidene camphor,
- Anisotriazine,
- Ethylhexyl Triazone,
- Diethylhexyl Butamido Triazone,
- la 2,4,6-tris-(4'-amino benzalmalonate de diisobutyle)-s-triazine,
- Benzophenone-3,
- Benzophenone-4,
- Benzophenone-5,
- 2-(4-diéthylamino-2-hydroxybenzoyl)benzoate de n-hexyle ;
- Methylene bis-Benzotriazolyl Tetramethylbutylphenol,
- Drometrizole Trisiloxane,
- Disodium Phenyl Dibenzimidazole Tetra-sulfonate,
et leurs mélanges.

25. Composition selon l'une quelconque des revendications 21 à 24, **caractérisée en ce qu'**elle comprend en outre des pigments ou des nanopigments d'oxydes métalliques enrobés ou non.

26. Composition selon l'une quelconque des revendications 21 à 25, **caractérisée en ce qu'**elle comprend des adjuvants cosmétiques choisis parmi les corps gras, les solvants organiques, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs anioniques, cationiques, amphotères ou non ioniques, les anti-inflammatoires, les antagonistes de substance P, les charges, les séquestrants, les polymères, les propulseurs, les agents alcalinisants ou acidifiants, les colorants et leurs mélanges.

27. Composition selon l'une quelconque des revendications 21 à 26, **caractérisée en ce qu'**elle se présente sous la forme d'émulsion, simple ou complexe, ou sous la forme d'un gel ou d'un gel-crème, de poudre, de bâtonnet solide, ou sous forme de mousse ou de spray.
